# EUROPEAN PATENT SPECIFICATION

(11) **EP 0 712 442 B1**
(45) Date of publication and mention of the grant of the patent: **27.03.2002**
(21) Application number: 94921762.4
(22) Date of filing: 29.07.1994
(51) Int. Cl.: C12N 15/62, C12N 15/31, C12N 15/54, C12N 1/21, A61K 38/45

(54) **VACCINE COMPOSITIONS**
REKOMBINANTE TETC-FUSIONSPROTEIN ENTHALTENE IMPFSTOFFZUSAMMENSETZUNGEN
COMPOSITIONS VACCINALES

(30) Priority: 30.07.1993 WO PCT/GB93/01617; 31.01.1994 GB 9401787
(43) Date of publication of application: 22.05.1996
(73) Proprietor: MEDEVA HOLDINGS B.V., 1078 ED Amsterdam (NL)
(72) Inventor: KHAN, Mohammed Anjam, Tennis Court Road Cambridge CB2 1QP (GB); HORMAECHE, Carlos Estenio, Tennis Court Road Cambridge CB2 1QP (GB); CHATFIELD, Steven Neville Medeva Vaccine Research, Science and Technology London SW7 2AY (GB); DOUGAN, Gordon Medeva Vaccine Research Unit Dept., Technology London SW7 2AY (GB)
(74) Representative: Woods, Geoffrey Corlett
(86) International application number: GB9401647
(87) International publication number: WO9504151

(56) References cited:
- EP-A- 0 432 965
- WO-A-89/06974
- WO-A-91/09621
- WO-A-92/15689
- WO-A-93/08290
- WO-A-94/03615
- BIO/TECHNOLOGY, vol.10, no.8, August 1992, NATURE AMERICA, INC., NEW YORK, US; pages 888 - 892 S.N. CHATFIELD ET AL. 'Use of the nirB promoter to direct the stable expression of heterologous antigens in Salmonella oral vaccine strains: Development of a single-dose oral tetanus vaccine'
- J. IMMUNOLOGY, vol.141, no.5, 1 September 1988, AM. SOC. IMMUNOLOGISTS,US; pages 1687 - 1694 C. AURIAULT ET AL. 'Analysis of T and B cell epitopes of the Schistosoma mansoni P28 antigen in the rat model by using synthetic peptides'
- NUCLEIC ACIDS REASEARCH, vol.19, no.11, 11 June 1991, IRL,OXFORD UNIVERSITY PRESS, UK; pages 2889 - 2892 M.D. OXER ET AL. 'High level heterologous expression in E. coli using the anaerobically-activated nirB promoter'
- FEMS SYMPOSIUM, NO. 73. BACTERIAL PROTEIN TOXINS; GUSTAV FISCHER VERLAG: STUTTGART, GERMANY; 0 (0). 1994 FREER, J. ET AL (ED.)

### SEQUENCE LISTING

(1) GENERAL INFORMATION:
   (i) APPLICANT:
      (A) NAME: MEDEVA HOLDINGS BV
      (B) STREET: CHURCHILL-LAAN 223
      (C) CITY: AMSTERDAM
      (E) COUNTRY: THE NETHERLANDS
      (F) POSTAL CODE (ZIP): 1078 ED
   (ii) TITLE OF INVENTION: VACCINES
   (iii) NUMBER OF SEQUENCES: 20
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: PCT/GB93/01617
      (B) FILING DATE: 30-JUL-1993
   (vi) PRIOR APPLICATION DATA:
      (A) APPLICATION NUMBER: GB 9401787.8
      (B) FILING DATE: 31-JAN-1994
(2) INFORMATION FOR SEQ ID NO: 1:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Escherichia coli
   (ix) FEATURE:
      (A) NAME/KEY: promoter
      (B) LOCATION: 1..61
   (xi) SEQUENCE DESCRIPTION: SEQ ID NO: 1:
   AATTCAGGTA AATTTGATGT ACATCAAATG GTACCCCTTG CTGAATCGTT AAGGTAGGCG 60
   GTAGGGCC 68
(2) INFORMATION FOR SEQ ID NO: 2:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 2:
   AATTCAGGTA AATTTGATGT ACATCAAATG GTACCCCTTG CTGAATCGTT AAGGTAGGCG 60
   GTAGGGCC 68
(2) INFORMATION FOR SEQ ID NO: 3:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (xi) SEQUENCE DESCRIPTION: SEQ ID NO: 3:
   GTCCATTTAA ACTACATGTA GTTTACCATG GGGAACGACT TAGCAATTCC ATCCGCCATC 60
(2) INFORMATION FOR SEQ ID NO: 4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 4:
   AAAGACTCCG CGGGCGAAGT T 21
(2) INFORMATION FOR SEQ ID NO: 5:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (xi) SEQUENCE DESCRIPTION: SEQ ID NO: 5:
   CTATGGATCC TTAACTAGTG ATTCTAGAGG GCCCCGGCCC GTCGTTGGTC CAACCTTCAT    60
   CGGT                                                                  64
(2) INFORMATION FOR SEQ ID NO: 6:
   (i) SEQUENCE CHARACTERISTICS:
      (A) LENGTH: 3754 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 6:
   TTCAGGTAAA TTTGATGTAC ATCAAATGGT ACCCCTTGCT GAATCGTTAA GGTAGGCGGT     60
   AGGGCCCAGA TCTTAATCAT CCACAGGAGA CTTTCTGATG AAAAACCTTG ATTGTTGGGT    120
   CGACAACGAA GAAGACATCG ATGTTATCCT GAAAAAGTCT ACCATTCTGA ACTTGGACAT    180
   CAACAACGAT ATTATCTCCG ACATCTCTGG TTTCAACTCC TCTGTTATCA CATATCCAGA    240
   TGCTCAATTG GTGCCGGGCA TCAACGGCAA AGCTATCCAC CTGGTTAACA ACGAATCTTC    300
   TGAAGTTATC GTGCACAAGG CCATGGACAT CGAATACAAC GACATGTTCA ACAACTTCAC    360
   CGTTAGCTTC TGGCTGCGCG TTCCGAAAGT TTCTGCTTCC CACCTGGAAC AGTACGGCAC    420
   TAACGAGTAC TCCATCATCA GCTCTATGAA GAAACACTCC CTGTCCATCG GCTCTGGTTG    480
   GTCTGTTTCC CTGAAGGGTA ACAACCTGAT CTGGACTCTG AAAGACTCCG CGGGCGAAGT    540
   TCGTCAGATC ACTTTCCGCG ACCTGCCGGA CAAGTTCAAC GCGTACCTGG CTAACAAATG    600
   GGTTTTCATC ACTATCACTA ACGATCGTCT GTCTTCTGCT AACCTGTACA TCAACGGCGT    660
   TCTGATGGGC TCCGCTGAAA TCACTGGTCT GGGCGCTATC CGTGAGGACA ACAACATCAC    720
   TCTTAAGCTG GACCGTTGCA ACAACAACAA CCAGTACGTA TCCATCGACA AGTTCCGTAT    780
   CTTCTGCAAA GCACTGAACC CGAAAGAGAT CGAAAAACTG TATACCAGCT ACCTGTCTAT    840
   CACCTTCCTG CGTGACTTCT GGGGTAACCC GCTGCGTTAC GACACCGAAT ATTACCTGAT    900
   CCCGGTAGCT TCTAGCTCTA AAGACGTTCA GCTGAAAAAC ATCACTGACT ACATGTACCT    960
   GACCAACGCG CCGTCCTACA CTAACGGTAA ACTGAACATC TACTACCGAC GTCTGTACAA   1020
   CGGCCTGAAA TTCATCATCA AACGCTACAC TCCGAACAAC GAAATCGATT CTTTCGTTAA   1080
   ATCTGGTGAC TTCATCAAAC TGTACGTTTC TTACAACAAC AACGAACACA TCGTTGGTTA   1140
   CCCGAAAGAC GGTAACGCTT TCAACAACCT GGACAGAATT CTGCGTGTTG GTTACAACGC   1200
   TCCGGGTATC CCGCTGTACA AAAAAATGGA AGCTGTTAAA CTGCGTGACC TGAAAACCTA   1260
   CTCTGTTCAG CTGAAACTGT ACGACGACAA AAACGCTTCT CTGGGTCTGG TTGGTACCCA   1320
   CAACGGTCAG ATCGGTAACG ACCCGAACCG TGACATCCTG ATCGCTTCTA ACTGGTACTT   1380
   CAACCACCTG AAAGACAAAA TCCTGGGTTG CGACTGGTAC TTCGTTCCGA CCGATGAAGG   1440
   TTGGACCAAC GACGGGCCGG GGCCCTCTAG AATCACTAGT TAAGGATCCG CTAGCCCGCC 1500
   TAATGAGCGG GCTTTTTTTT CTCGGGCAGC GTTGGGTCCT GGCCACGGGT GCGCATGATC 1560
   GTGCTCCTGT CGTTGAGGAC CCGGCTAGGC TGGCGGGGTT GCCTTACTGG TTAGCAGAAT 1620
   GAATCACCGA TACGCGAGCG AACGTGAAGC GACTGCTGCT GCAAAACGTC TGCGACCTGA 1680
   GCAACAACAT GAATGGTCTT CGGTTTCCGT GTTTCGTAAA GTCTGGAAAC GCGGAAGTCA 1740
   GCGCTCTTCC GCTTCCTCGC TCACTGACTC GCTGCGCTCG GTCGTTCGGC TGCGGCGAGC 1800
   GGTATCAGCT CACTCAAAGG CGGTAATACG GTTATCCACA GAATCAGGGG ATAACGCAGG 1860
   AAAGAACATG TGAGCAAAAG GCCAGCAAAA GGCCAGGAAC CGTAAAAAGG CCGCGTTGCT 1920
   GGCGTTTTTC CATAGGCTCC GCCCCCCTGA CGAGCATCAC AAAAATCGAC GCTCAAGTCA 1980
   GAGGTGGCGA AACCCGACAG GACTATAAAG ATACCAGGCG TTTCCCCCTG GAAGCTCCCT 2040
   CGTGCGCTCT CCTGTTCCGA CCCTGCCGCT TACCGGATAC CTGTCCGCCT TTCTCCCTTC 2100
   GGGAAGCGTG GCGCTTTCTC AATGCTCACG CTGTAGGTAT CTCAGTTCGG TGTAGGTCGT 2160
   TCGCTCCAAG CTGGGCTGTG TGCACGAACC CCCCGTTCAG CCCGACCGCT GCGCCTTATC 2220
   CGGTAACTAT CGTCTTGAGT CCAACCCGGT AAGACACGAC TTATCGCCAC TGGCAGCAGC 2280
   CACTGGTAAC AGGATTAGCA GAGCGAGGTA TGTAGGCGGT GCTACAGAGT TCTTGAAGTG 2340
   GTGGCCTAAC TACGGCTACA CTAGAAGGAC AGTATTTGGT ATCTGCGCTC TGCTGAAGCC 2400
   AGTTACCTTC GGAAAAAGAG TTGGTAGCTC TTGATCCGGC AAACAAACCA CCGCTGGTAG 2460
   CGGTGGTTTT TTTGTTTGCA AGCAGCAGAT TACGCGCAGA AAAAAAGGAT CTCAAGAAGA 2520
   TCCTTTGATC TTTTCTACGG GGTCTGACGC TCAGTGGAAC GAAAACTCAC GTTAAGGGAT 2580
   TTTGGTCATG AGATTATCAA AAAGGATCTT CACCTAGATC CTTTTAAATT AAAAATGAAG 2640
   TTTTAAATCA ATCTAAAGTA TATATGAGTA AACTTGGTCT GACAGTTACC AATGCTTAAT 2700
   CAGTGAGGCA CCTATCTCAG CGATCTGTCT ATTTCGTTCA TCCATAGTTG CCTGACTCCC 2760
   CGTCGTGTAG ATAACTACGA TACGGGAGGG CTTACCATCT GGCCCCAGTG CTGCAATGAT 2820
   ACCGCGAGAC CCACGCTCAC CGGCTCCAGA TTTATCAGCA ATAAACCAGC CAGCCGGAAG 2880
   GGCCGAGCGC AGAAGTGGTC CTGCAACTTT ATCCGCCTCC ATCCAGTCTA TTAATTGTTG 2940
   CCGGGAAGCT AGAGTAAGTA GTTCGCCAGT TAATAGTTTG CGCAACGTTG TTGCCATTGC 3000
   TGCAGGCATC GTGGTGTCAC GCTCGTCGTT TGGTATGGCT TCATTCAGCT CCGGTTCCCA 3060
   ACGATCAAGG CGAGTTACAT GATCCCCCAT GTTGTGCAAA AAAGCGGTTA GCTCCTTCGG 3120
   TCCTCCGATC GTTGTCAGAA GTAAGTTGGC CGCAGTGTTA TCACTCATGG TTATGGCAGC 3180
   ACTGCATAAT TCTCTTACTG TCATGCCATC CGTAAGATGC TTTTCTGTGA CTGGTGAGTA 3240
   CTCAACCAAG TCATTCTGAG AATAGTGTAT GCGGCGACCG AGTTGCTCTT GCCCGGCGTC 3300
   AACACGGGAT AATACCGCGC CACATAGCAG AACTTTAAAA GTGCTCATCA TTGGAAAACG 3360
   TTCTTCGGGG CGAAAACTCT CAAGGATCTT ACCGCTGTTG AGATCCAGTT CGATGTAACC 3420
   CACTCGTGCA CCCAACTGAT CTTCAGCATC TTTTACTTTC ACCAGCGTTT CTGGGTGAGC 3480
   AAAAACAGGA AGGCAAAATG CCGCAAAAAA GGGAATAAGG GCGACACGGA AATGTTGAAT 3540
   ACTCATACTC TTCCTTTTTC AATATTATTG AAGCATTTAT CAGGGTTATT GTCTCATGAG 3600
   CGGATACATA TTTGAATGTA TTTAGAAAAA TAAACAAATA GGGGTTCCGC GCACATTTCC 3660
   CCGAAAAGTG CCACCTGACG TCTAAGAAAC CATTATTATC ATGACATTAA CCTATAAAAA 3720
   TAGGCGTATC ACGAGGCCCT TTCGTCTTCA AGAA 3754
(2) INFORMATION FOR SEQ ID NO: 7:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 7:
   TAGTCTAGAA TGGCTGGCGA GCATATCAAG 30
(2) INFORMATION FOR SEQ ID NO: 8:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (xi) SEQUENCE DESCRIPTION: SEQ ID NO: 8:
   TTAGGATCCT TAGAAGGGAG TTGCAGGCCT                                    30
(2) INFORMATION FOR SEQ ID NO: 9:
   (i) SEQUENCE CHARACTERISTICS:
      (A) LENGTH: 4378 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 9:
(2) INFORMATION FOR SEQ ID NO: 10:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 10:
   AAAGACTCCG CGGGCGAAGT T
(2) INFORMATION FOR SEQ ID NO: 11:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (xi) SEQUENCE DESCRIPTION: SEQ ID NO: 11:
   TTATCTAGAG TCGTTGGTCC AACCTTCATC
(2) INFORMATION FOR SEQ ID NO: 12:
   (i) SEQUENCE CHARACTERISTICS:
      (A) LENGTH: 4366 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 12:

## Description

This invention relates to DNA constructs, replicable expression vectors containing the constructs, bacteria containing the constructs and vaccines containing the bacteria or fusion proteins expressed therefrom. More particularly, the invention relates to novel DNA constructs encoding the C-fragment of tetanus toxin, and to fusion proteins containing tetanus toxin C-fragment.

It is known to prepare DNA constructs encoding two or more heterologous proteins with a view to expressing the proteins in a suitable host as a single fusion protein. However, it has often been found that fusing two proteins together in this way leads to an incorrectly folded chimaeric protein which no longer retains the properties of the individual components. For example, the B-subunits of the Vibrio cholerae (CT-B) and E. coli (LT-B) enterotoxins are powerful mucosal immunogens but genetic fusions to these subunits can alter the structure and properties of the carriers and hence their immunogenicity (see M. Sandkvist et al. J. Bacteriol. 169, pp4570-6, 1987, Clements et al. 1990 and M. Lipscombe et al. Mol. Microbiol. 5, pp 1385, 1990). Moreover, many heterologous proteins expressed in bacteria are not produced in soluble properly folded or active forms and tend to accumulate as insoluble aggregates (see C. Schein et al. Bio/Technology 6, pp 291-4, 1988 and R. Halenbeck et al. Bio/Technology 7, pp 710-5, 1989.

In our earlier unpublished international patent application PCT/GB93/01617, it is disclosed that by providing a DNA sequence encoding tetanus toxin C-fragment (TetC) linked via a "hinge region" to a second sequence encoding an antigen, the expression of the sequence in bacterial cells is enhanced relative to constructs wherein the C-fragment is absent. For example, the expression level of the full length P28 glutathione *S*-tranferase protein of S. mansoni when expressed as a fusion to TetC from the nirB promoter was greater than when the P28 protein was expressed alone from the nirB promoter. The TetC fusion to the full length P28 protein of S. mansoni was soluble and expressed in both E. coli and S. typhimurium. In addition, the TetC-P28 fusion protein was capable of being affinity purified by a glutathione agarose matrix, suggesting that the P28 had folded correctly to adopt a conformation still capable of binding to its natural substrate. It was previously considered that a hinge region, which typically is a sequence encoding a high proportion of proline and/or glycine amino acids, is essential for promoting the independent folding of both the TetC and the antigenic protein fused thereto. However, it has now been discovered, surprisingly in view of the previous studies on CT-B and LT-B referred to above, that when the hinge region is omitted between the TetC and a second antigen such as P28, the proteins making up the fusion do exhibit correct folding as evidenced by affinity purification on a glutathione agarose matrix.

Accordingly, in a first aspect, the invention provides a DNA construct comprising a DNA sequence encoding a fusion protein of the formula TetC-(Z)ₐ-Het, wherein TetC is the C fragment of tetanus toxin; Het is a heterologous protein; Z is an amino acid, and a is less than 4, provided that (Z)ₐ does not include the sequence Gly-Pro.

In one embodiment (Z)ₐ is a chain of two or three amino acids, the DNA sequence for which defines a restriction endonuclease cleavage site.

In another embodiment, a is zero.

Generally, the group (Z)ₐ will not contain either glycine or proline at all.

The group (Z)ₐ may be a chain of amino acids substantially devoid of biological activity.

In a second aspect the invention provides a replicable expression vector, for example suitable for use in bacteria, containing a DNA construct as hereinbefore defined.

In another aspect, the invention provides a host (e.g. a bacterium) containing a DNA construct as hereinbefore defined, the DNA construct being present in the host either in the form of a replicable expression vector such as a plasmid, or being present as part of the host chromosome, or both.

In a further aspect, the invention provides a fusion protein of the form TetC-(Z)ₐ-Het as hereinbefore defined, preferably in substantially pure form, said fusion protein being expressible by a replicable expression vector as hereinbefore defined.

In a further aspect the invention provides a process for the preparation of a bacterium (preferably an attenuated bacterium) which process comprises transforming a bacterium (e.g. an attenuated bacterium) with a DNA construct as hereinbefore defined.

The invention also provides a vaccine composition comprising an attenuated bacterium, or a fusion protein, as hereinbefore defined, and a pharmaceutically acceptable carrier.

The heterologous protein "Het" may for example be a heterologous antigenic sequence, e.g. an antigenic sequence derived from a virus, bacterium, fungus, yeast or parasite.

Examples of viral antigenic sequences are sequences derived from a type of human immunodeficiency virus (HIV) such as HIV-1 or HIV-2, the CD4 receptor binding site from HIV, for example from HIV-1 or -2., hepatitis A, B or C virus, human rhinovirus such as type 2 or type 14, Herpes simplex virus, poliovirus type 2 or 3, foot-and-mouth disease virus (FMDV), rabies virus, rotavirus, influenza virus, coxsackie virus, human papilloma virus (HPV), for example the type 16 papilloma virus, the E7 protein thereof, and fragments containing the E7 protein or its epitopes; and simian immunodeficiency virus (SIV).

Examples of antigens derived from bacteria are those derived from Bordetella pertussis (e.g. P69 protein and filamentous haemagglutinin (FHA) antigens), Vibrio cholerae, Bacillus anthracis, and E.coli antigens such as E.coli heat Labile toxin B subunit (LT-B), E.coli K88 antigens, and enterotoxigenic E.coli antigens. Other examples of antigens include the cell surface antigen CD4, Schistosoma mansoni P28 glutathione *S*-transferase antigens (P28 antigens) and antigens of flukes, mycoplasma, roundworms, tapeworms, Chlamydia trachomatis, and malaria parasites, eg. parasites of the genus plasmodium or babesia, for example Plasmodium falciparum, and peptides encoding immunogenic epitopes from the aforementioned antigens.

Particular antigens include the full length Schistosoma mansoni P28, and oligomers (e.g. 2, 4 and 8-mers) of the immunogenic P28 aa 115-131 peptide (which contains both a B and T cell epitope), and human papilloma virus E7 protein, Herpes simplex antigens, foot and mouth disease virus antigens and simian immunodeficiency virus antigens.

The DNA constructs of the present invention may contain a promoter whose activity is induced in response to a change in the surrounding environment. An example of such a promoter sequence is one which has activity which is induced by anaerobic conditions. A particular example of such a promoter sequence is the nirB promoter which has been described, for example in International Patent Application PCT/GB92/00387. The nirB promoter has been isolated from E.coli, where it directs expression of an operon which includes the nitrite reductase gene nirB (Jayaraman et al, J. Mol. Biol. 196, 781-788, 1987), and nirD, nirC, cysG (Peakman et al, Eur. J. Biochem. 191, 315323, 1990). It is regulated both by nitrite and by changes in the oxygen tension of the environment, becoming active when deprived of oxygen, (Cole, Biochem, Biophys. Acta. 162, 356-368, 1968). Response to anaerobiosis is mediated through the protein FNR, acting as a transcriptional activator, in a mechanism common to many anaerobic respiratory genes. By deletion and mutational analysis the part of the promoter which responds solely to anaerobiosis has been isolated and by comparison with other anaerobically regulated promoters a consensus FNR-binding site has been identified (Bell et al, Nucl, Acids. Res. 17, 3865-3874, 1989; Jayaraman et al, Nucl, Acids, Res. 17, 135-145, 1989). It has also been shown that the distance between the putative FNR-binding site and the -10 homology region is critical (Bell et al, Molec. Microbiol.4, 1753-1763, 1990). It is therefore preferred to use only that part of the nirB promoter which responds solely to anaerobiosis. As used herein, references to the nirB promoter refer to the promoter itself or a part or derivative thereof which is capable of promoting expression of a coding sequence under anaerobic conditions. The preferred sequence, and which contains the nirB promoter is:

In a most preferred aspect, the present invention provides a DNA molecule comprising the nirB promoter operably linked to a DNA sequence encoding a fusion protein as hereinbefore defined.

In another preferred aspect of the invention, there is provided a replicable expression vector, suitable for use in bacteria, containing the nirB promoter sequence operably linked to a DNA sequence encoding a fusion protein as hereinbefore defined.

The DNA molecule or construct may be integrated into the bacterial chromosome, e.g. by methods known per se, and thus in a further aspect, the invention provides a bacterium having in its chromosome, a DNA sequence or construct as hereinbefore defined.

Stable expression of the fusion protein can be obtained in vivo. The fusion protein can be expressed in an attenuated bacterium which can thus be used as a vaccine.

The attenuated bacterium may be selected from the genera Salmonella, Bordetella, Vibrio, Haemophilus, Neisseria and Yersinia. Alternatively, the attenuated bacterium may be an attenuated strain of enterotoxigenic Escherichia coli. In particular the following species can be mentioned: S.typhi - the cause of human typhoid; S.typhimurium - the cause of salmonellosis in several animal species; S.enteritidis - a cause of food poisoning in humans; S.choleraesuis - a cause of salmonellosis in pigs; Bordetella pertussis - the cause of whooping cough; Haemophilus influenzae - a cause of meningitis; Neisseria gonorrhoea the cause of gonorrhoea; and Yersinia - a cause of food poisoning.

Examples of attenuated bacteria are disclosed in, for example EP-A-0322237 and EP-A-0400958, the disclosures in which are incorporated by reference herein.

An attenuated bacterium containing a DNA construct according to the invention, either present in the bacterial chromosome, or in plasmid form, or both, can be used as a vaccine. Fusion proteins (preferably in substantially pure form) expressed by the bacteria can also be used in the preparation of vaccines. For example, a purified TetC-P28 fusion protein in which the TetC protein is linked via its C-terminus to the P28 protein with no intervening hinge region has been found to be immunogenic on its own. In a further aspect therefore, the invention provides a vaccine composition comprising a pharmaceutically acceptable carrier or diluent and, as active ingredient, an attenuated bacterium or fusion protein as hereinbefore defined.

The vaccine may comprise one or more suitable adjuvants.

The vaccine is advantageously presented in a lyophilised form, for example in a capsular form, for oral administration to a patient. Such capsules may be provided with an enteric coating comprising, for example, Eudragit "S", Eudragit "L", Cellulose acetate, Cellulose acetate phthalate or Hydroxypropylmethyl Cellulose. These capsules may be used as such, or alternatively, the lyophilised material may be reconstituted prior to administration, e.g. as a suspension. Reconstitution is advantageously effected in buffer at a suitable pH to ensure the viability of the organisms. In order to protect the attenuated bacteria and the vaccine from gastric acidity, a sodium bicarbonate preparation is advantageously administered before each administration of the vaccine. Alternatively, the vaccine may be prepared for parenteral administration, intranasal administration or intramammary administration.

The attenuated bacterium containing the DNA construct or fusion protein of the invention may be used in the prophylactic treatment of a host, particularly a human host but also possibly an animal host. An infection caused by a microorganism, especially a pathogen, may therefore be prevented by administering an effective dose of an attenuated bacterium according to the invention. The bacterium then expresses the fusion protein which is capable of raising antibody to the micro-organism. The dosage employed will be dependent on various factors including the size and weight of the host, the type of vaccine formulated and the nature of the fusion protein.

An attenuated bacterium according to the present invention may be prepared by transforming an attenuated bacterium with a DNA construct as hereinbefore defined. Any suitable transformation technique may be employed, such as electroporation. In this way, an attenuated bacterium capable of expressing a protein or proteins heterologous to the bacterium may be obtained. A culture of the attenuated bacterium may be grown under aerobic conditions. A sufficient amount of the bacterium is thus prepared for formulation as a vaccine, with minimal expression of the fusion protein occurring.

The DNA construct may be a replicable expression vector comprising the nirB promoter operably linked to a DNA sequence encoding the fusion protein. The nirB promoter may be inserted in an expression vector, which already incorporates a gene encoding one of the heterologous proteins (e.g. the tetanus toxin C fragment), in place of the existing promoter controlling expression of the protein. The gene encoding the other heterologous protein (e.g. an antigenic sequence) may then be inserted. The expression vector should, of course, be compatible with the attenuated bacterium into which the vector is to be inserted.

The expression vector is provided with appropriate transcriptional and translational control elements including, besides the nirB promoter, a transcriptional termination site and translational start and stop codons. An appropriate ribosome binding site is provided. The vector typically comprises an origin of replication and, if desired, a selectable marker gene such as an antibiotic resistance gene. The vector may be a plasmid.

The invention will now be illustrated but not limited, by reference to the following examples and the accompanying drawings, in which:
Figure 1 is a schematic illustration of the construction of plasmid pTECH1;

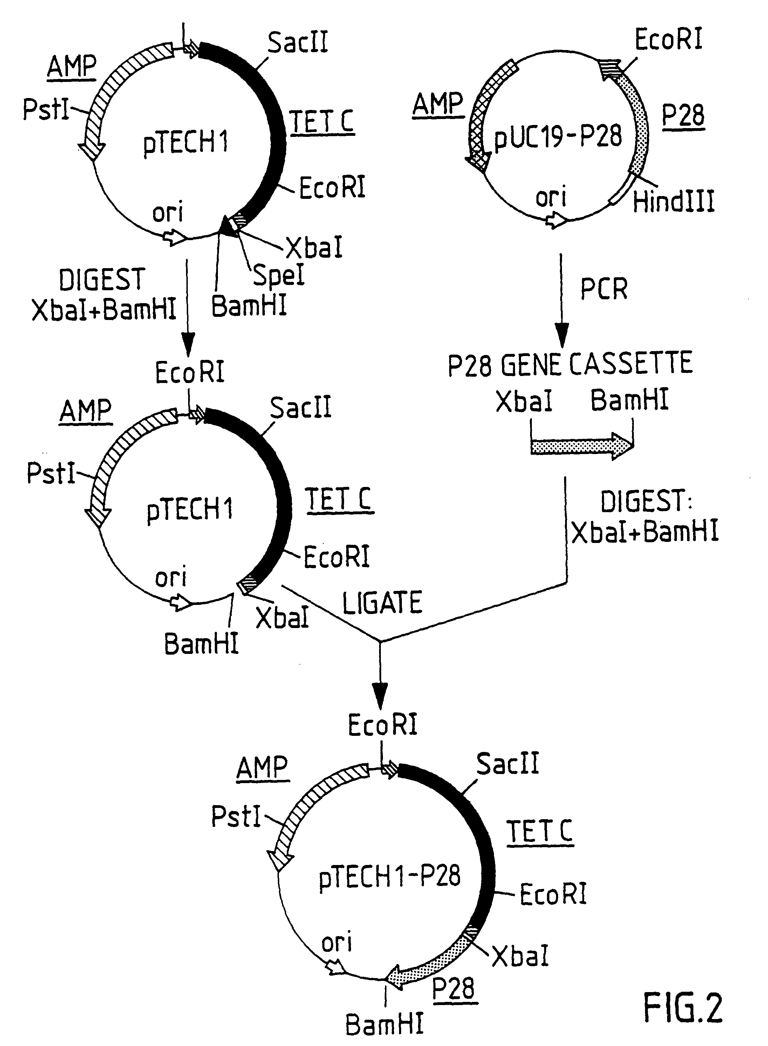

Figure 2 illustrates schematically the preparation of the plasmid pTECH1-28 from the starting materials pTECH1 and PUC19-P28;

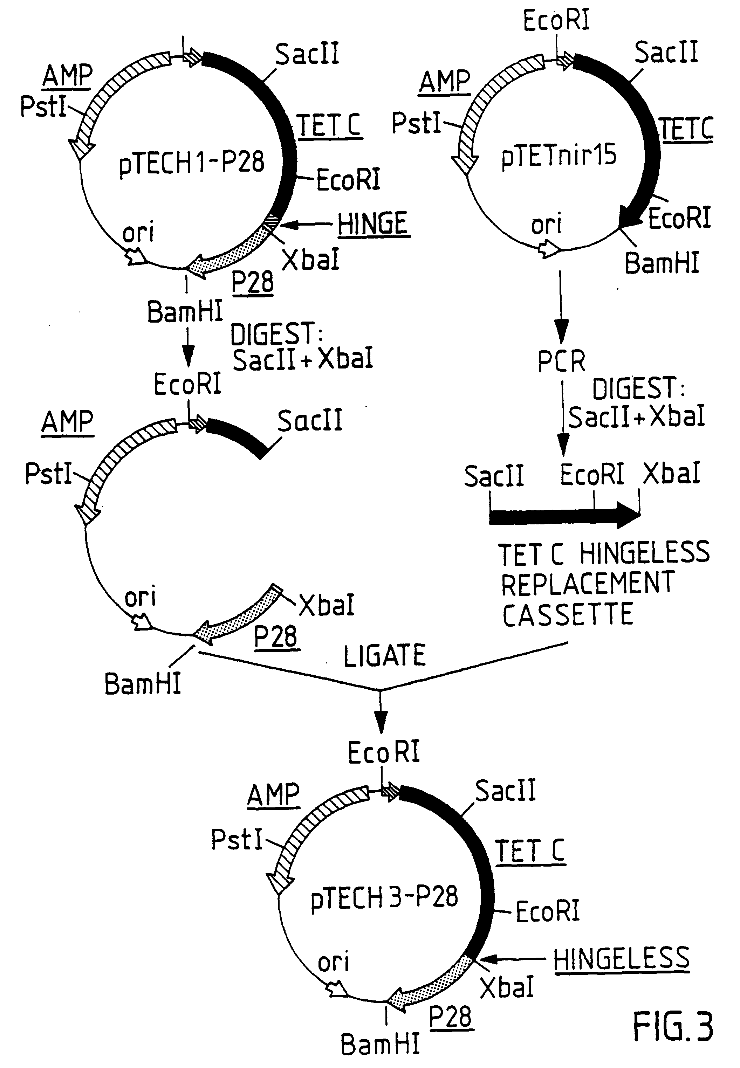

Figure 3 illustrates schematically the preparation of the plasmid pTECH3-P28 from the starting materials plasmids PTECH1-P28 and pTETnir15;
Figures 4 and 5 are western blots obtained from bacterial cells harbouring the pTECH3-P28 construct; and
Figure 6 illustrates the glutathione affinity purification of TetC fusions as determined by SDS-PAGE and Coomassie Blue Staining.

In accordance with the invention a vector was constructed to allow genetic fusions to the C-terminus of the highly immunogenic C fragment of tetanus toxin, without the use of a heterologous hinge domain. A fusion was constructed, with the gene encoding the protective 28kDa glutathione *S*-transferase from Schistosoma mansoni. The recombinant vector was transformed into Salmonella typhimurium (SL338; rm⁺). The resulting chimeric protein was stably expressed in a soluble form in salmonella as assessed by western blotting with fragment C and glutathione *S*-transferase antisera. Furthermore it was found that the P28 component of the fusion retains the capacity to bind glutathione.

The construction of the vector and the properties of the fusion protein expressed therefrom are described in more detail below.

### EXAMPLE 1

### Preparation of pTECH1

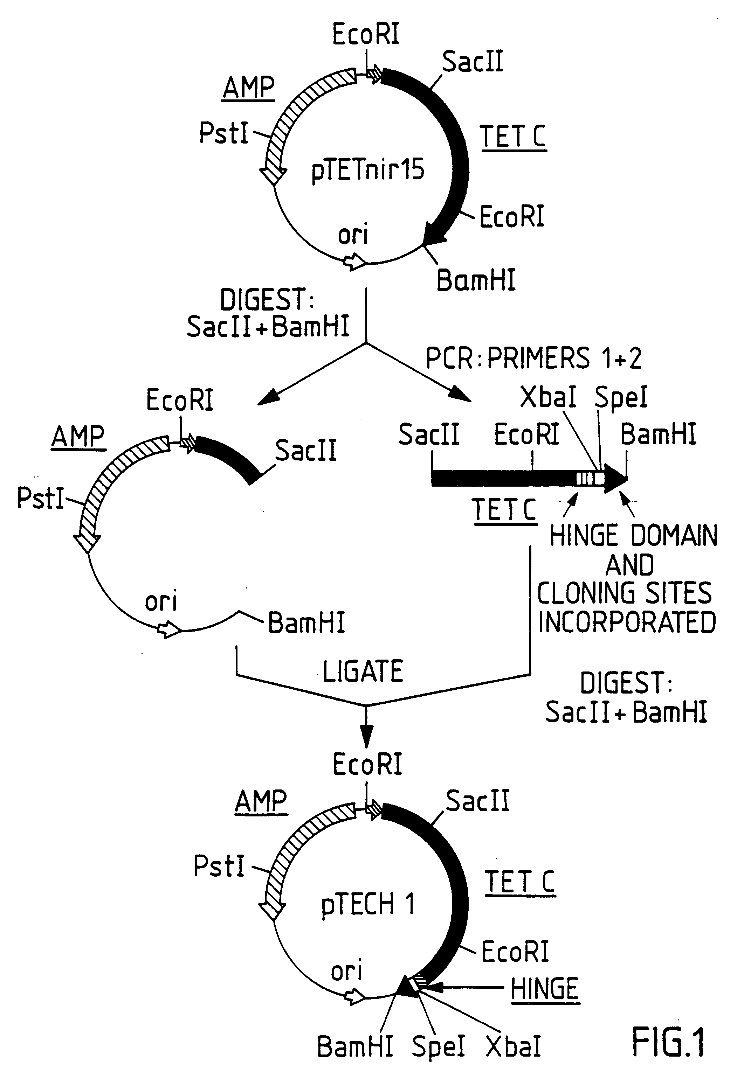

The preparation of pTECH1, a plasmid incorporating the nirB promoter and TetC gene, and a DNA sequence encoding a hinge region and containing restriction endonuclease sites to allow insertion of a gene coding for a second or guest protein, is illustrated in Figure 1. Expression plasmid pTETnir15, the starting material shown in Figure 1, was constructed from pTETtac115 (Makoff *et al,* Nucl. Acids Res. 17 10191-10202, 1989); by replacing the EcoRI-ApaI region (1354bp) containing the lacI gene and tac promoter with the following pair of oligos 1 and 2:

The oligonucleotides were synthesised on a Pharmacia Gene Assembler and the resulting plasmids confirmed by sequencing (Makoff *et al,* Bio/Technology 7, 1043-1046, 1989).

The pTETnir15 plasmid was then used for construction of the pTECH1 plasmid incorporating a polylinker region suitable as a site for insertion of heterologous DNA to direct the expression of fragment C fusion proteins. pTETnir15 is a known pAT153-based plasmid which directs the expression of fragment C. However, there are no naturally occurring convenient restriction sites present at the 3'-end of the TetC gene. Therefore, target sites, preceded by a hinge region, were introduced at the 3'-end of the TetC coding region by means of primers SEQ ID NO: 4 and SEQ ID NO: 5 tailored with "add-on" adapter sequences (Table 1), using the polymerase chain reaction (PCR) [K. Mullis *et al*, Cold Spring Harbor Sym. Quant. Biol. 51, 263-273 1986]. Accordingly, pTETnir15 was used as a template in a PCR reaction using primers corresponding to regions covering the SacII and BamHI sites. The anti-sense primer in this amplification was tailored with a 38 base 5'-adaptor sequence. The anti-sense primer was designed so that a sequence encoding novel XbaI, SpeI and BamHI sites were incorporated into the PCR product. In addition, DNA sequences encoding additional extra amino acids including proline were incorporated (the hinge regions) and a translation stop codon signal in frame with the fragment C open reading frame.

The PCR product was gel-purified and digested with SacII and BamHI, and cloned into the residual 2.8 kb vector pTETnir15 which had previously been digested by SacII and BamHI. The resulting plasmid purified from transformed colonies and named pTECH 1 is shown in Figure 1. Heterologous sequences such as the sequence encoding the Schistosoma mansoni P28 glutathione *S*-transferase (P28) were cloned into the XbaI SpeI and BamHI sites in accordance with known methods.

The DNA sequence of the plasmid pTECH1 is shown in the sequence listing as SEQ ID NO: 6.

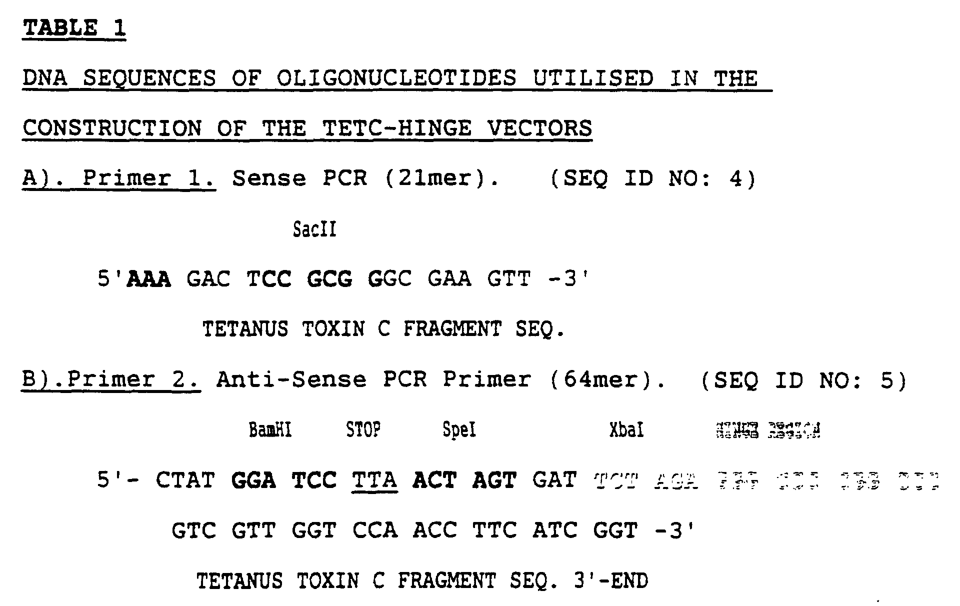

### EXAMPLE 2

### Construction of PTECH1-P28

A P28 gene expression cassette was produced by PCR using pUC19-P28 DNA (a kind gift from Dr R Pierce, Pasteur Institute, Lille) as template. Oligonucleotide primers were designed to amplify the full length P28 gene beginning with the start codon and terminating with the stop codon. In addition, the sense and antisense primers were tailored with the restriction sites for XbaI and BamHI respectively. The primers are shown in the sequence listing as SEQ ID NO: 7 and SEQ ID NO: 8.
The product was gel-purified and digested with XbaI and BamHI and then cloned into pTECH1 which had previously been digested with these enzymes and subsequently gel-purified. The DNA sequence of pTECH1 - P28 is shown in sequence listing as SEQ ID NO: 9.

### Expression of the TetC-Hinge-P28 fusion protein

Several bacterial strains, namely S. typhimirium strains SL 5338 (A. Brown et al, J.Infect.Dis. 155, 86-92, 1987) and SL3261 and E. coli (TG2) were transformed with pTECH1-P28 by means of electroporation. SL3261 strains harbouring the pTECH1-P28 plasmid have been deposited at the National Collection of Type Cultures, 61 Colindale Avenue, London, NW9 5HT, UK under the accession number NCTC 12833. A strain of SL3261 containing the pTECH1 plasmid has been deposited under accession number NCTC 12831. The identity of recombinants was verified by restriction mapping of the plasmid DNA harboured by the cells. Further expression of the TetC-P28 fusion protein was then evaluated by SDS-PAGE and western blotting of bacterial cells harbouring the construct It was found that the fusion protein remains soluble, cross-reacts with antisera to both TetC and P28, and is also of the expected molecular weight, 80kDa1, for a full length fusion.

The fusion protein was stably expressed in E.coli (TG2) and S. typhimurium (SL5338,SL3261) as judged by SDS-PAGE and western blotting. Of interest was a band of 50kDal which co-migrates with the TetC-Hinge protein alone and cross-reacts exclusively with the anti-TetC sera is visible in a western blot. As the codon selection in the hinge region has been designed to be suboptimal, the rare codons may cause pauses during translation which may occasionally lead to the premature termination of translation, thus accounting for this band.

### Affinity purification of the TetC-P28 fusion

Glutathione is the natural substrate for P28, a glutathione *S*-transferase. The amino acid residues involved in binding glutathione are thought to be spatially separated in the primary structure of the polypeptide and brought together to form a glutathione binding pocket in the tertiary structure (P. Reinemer *et al.* EMBO, J8, 1997-2005, 1991). In order to gauge whether the P28 component of the fusion has folded correctly to adopt a conformation capable of binding glutathione, its ability to be affinity purified on a glutathione-agarose matrix was tested. The results obtained (not shown) demonstrated that TetC-P28 can indeed bind to the matrix and the binding is reversible, as the fusion can be competitively eluted with free glutathione.

### EXAMPLE 3

### Construction of pTECH3-P28

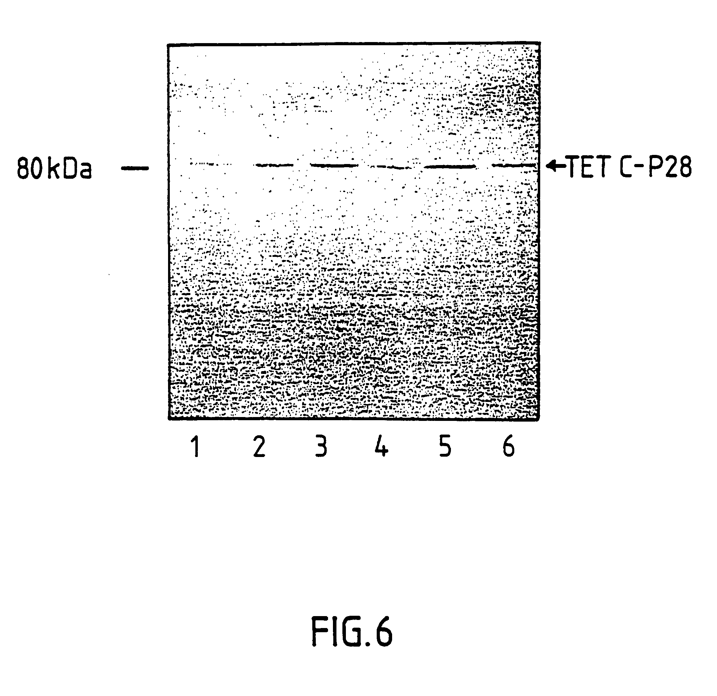

The plasmid pTECH1-P28 directs the expression of the S. mansoni P28 protein as a C-terminal fusion to fragment C from tetanus toxin separated by a heterologous hinge domain. Expression of the fusion protein is under the control of the nirB promoter. The vector pTECH3-P28 was in part constructed from the plasmid pTETnir15 by the polymerase chain reaction (PCR) using the high fidelity thermostable DNA polymerase from Pyrococcus fusorius, which possesses an associated 3'5' exonuclease proofreading activity. The sequence of steps is summarised in Figure 5. In order to generate a TetC-hingeless replacement cassette, the segment of DNA from the unique SacII site within the TetC gene to the final codon was amplified by means of the PCR reaction, using pTETnir15 as template DNA. The primers used in the PCR amplification are shown in the sequence listing as SEQ ID NO: 10 and SEQ ID NO: 11. The antisense primer in this amplification reaction was tailored with an XbaI recognition sequence.

The amplification reaction was performed according to the manufacturer's instructions (Stratagene, La Jolla, CA, USA). The product was gel-purified, digested with SacII and XbaI, and then cloned into the residual pTECH1-P28 vector which had been previously digested with the respective enzymes SacII and XbaI. The resulting vector was designated pTECH3-P28. The DNA sequence of pTECH3-P28 is shown in the sequenc listing as SEQ ID NO: 12.

### EXAMPLE 4

### Transformation of S. typhimurium SL5338 (galE r⁻m⁺) with pTECH3-F28, and Analysis of the Transformants

S. typhimurium SL5338 (galE r⁻m⁺) were cultured in either L or YT broth and on L-agar with ampicillin (50 g/ml) if appropriate and were transformed with the pTECH3-P28 plasmid. The transformation protocol was based on the method described by MacLachlan and Sanderson. (MacLachlan PR and Sanderson KE, 1985. Transformation of Salmonella typhimurium with plasmid DNA : differences between rough and smooth strains. J. Bacteriology 161, 442-445).

A 1ml overnight culture of S. typhimurium SL5338 (r⁻m⁺; Brown A, Hormaeche CE, Demarco de Hormaeche R, Dougan G, Winther M, Maskell D, and Stocker BAD, 1987. J. Infect.Dis. 155, 86-92) was used to inoculate 100 ml of LB broth and shaken at 37°C until the culture reached OD₆₅₀ = 0.2. The cells were harvested at 3000 x g and resuspended in 0.5 volumes if ice-cold 0.1M MgCl₂. The cells were pelleted again and resuspended in 0.5 volumes of ice-cold CaCl₂. This step was repeated once more and the cells resuspended in 1 ml of 0.1M CaCl₂ to which was added 50 µl of TES (50 mM Tris, 10 mM EDTA, 50 mM NaCl, pH 8.0). The cells were incubated on ice for 45 to 90 minutes. To 150µl of cells was added 100ng of plasmid DNA in 1 - 2µl. The mixture was incubated on ice for 30 minutes prior to heat-shock at 42°C for 2 minutes, and immediate reincubation on ice for 1 minute. To the transformed mixture was added 2 ml of LB broth and incubated for 1.5 hours to allow expression of the ampicillin drug resistance gene, B-lactamase. Following incubation 20 µl and 200 µl of cells were spread on to LB agar plates containing 50 µg/ml of ampicillin. The plates were dried and incubated at 37°C overnight.

The identity of recombinants was verified by restriction mapping of the plasmid DNA and by western blotting with antisera directed against TetC and P28.

### SDS-PAGE and Western Blotting

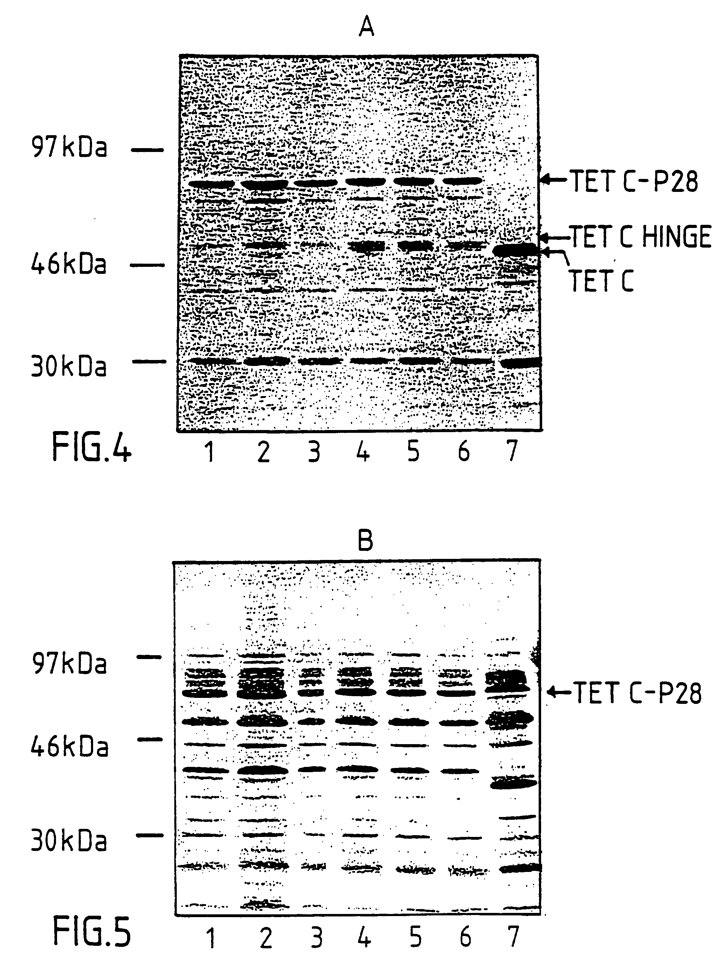

Expression of the TetC fusions was tested by SDS-PAGE and western blotting. S. typhimurium SL5338 (galE r⁻m⁺) bacterial cells containing the pTECH3-P28 plasmid and growing in mid-log phase, with antibiotic selection, were harvested by centrifugation and the proteins fractionated by 10% SDS-PAGE. The proteins were transferred to a nitrocellulose membrane by electroblotting and reacted with either a polyclonal rabbit antiserum directed against TetC or the full length P28 protein. The blots were then probed with goat anti-rabbit Ig conjugated to horse-radish peroxidase (Dako, High Wycombe, Bucks, UK) and developed with 4-chloro-1-napthol). The results of the western blotting experiments are shown in Figures 4 and 5; Figure 4 illustrating the results of probing with rabbit anti-TetC polyclonal antiserum and Figure 5 illustrates the results of probing with rabbit anti-P28 polyclonal antiserum. In each case lanes 1, 2 and 3 are independent clones of SL5338 (pTECH3-P28), lanes 4, 5 and 6 are SL5338 (pTECH1-P28) and lane 7 is SL5338 (pTETnirl5). The molecular weight markers are indicated. From the results, it is evident that the fusion protein remains soluble, reacts with antisera to both TetC and P28, and is also of the expected molecular weight, 80 kDal, for a full length fusion (Figure 4). Furthermore the fusion protein appears to be stably expressed.

### Glutathione-Agarose Affinity Purification

Glutathione is the natural substrate for P28, a glutathione *S*-transferase. The amino acid residues involved in binding glutathione are thought to be spatially separated in the primary structure of the polypeptide and brought together to form a glutathione binding pocket in the tertiary structure. In order to gauge whether the P28 component of the fusion has folded correctly to adopt a conformation capable of binding glutathione, we tested its ability to be affinity purified on a glutathione agarose matrix.

Bacterial cells containing pTECH3-P28 and expressing the TetC full length P28 gene fusion were grown to log phase, chilled on ice, and harvested by centrifugation at 2500 x g for 15 min at 4C. The cells were resuspended in 1/15th the original volume of ice-cold phosphate buffered saline (PBS) and lysed by sonication in a MSE Soniprep 150 (Gallenkamp, Leicester, UK). The insoluble material was removed by centrifugation and to the supernatant was added 1/6 volume of a 50% slurry of pre-swollen glutathione-agarose beads (Sigma, Poole, Dorset, UK). After mixing gently at room temperature for 1 hour the beads were collected by centrifugation at 1000 x g for 10 secs. The supernatant was discarded and the beads resuspended in 20 volumes of cold PBS-0.5% Triton X100 and the beads collected again by centrifugation. The washing step was repeated three more times. The fusion protein was eluted by adding 1 volume of SDS-PAGE sample buffer. For comparison purposes, a similar procedure was followed with bacterial cells containing the PTECH1-P28 plasmid from which TetC-hinge-P28 fusion protein is expressed. Extracts from clones containing either plasmid were compared using SDS-PAGE and the results are shown in Figure 6. In Figure 6, lanes 1, 2 and 3 are clones of SL5338 (pTECH1-P28) whereas lanes 4, 5 and 6 are independent clones of SL 5338 (pTECH3-P28).

The results suggest that the TetC-P28 fusion protein can indeed bind to the matrix and the binding is reversible regardless of the absence of a heterologous hinge domain (data not shown) It is possible that a peptide sequence present at the C-terminus of TetC may in fact impart flexibility to this particular region.

## Claims

1. A DNA construct comprising a DNA sequence encoding a fusion protein of the formula TetC-(Z)ₐ-Het, wherein TetC is the C fragment of tetanus toxin, Het is a heterologous protein, Z is an amino acid, and a is less than 4, provided that (Z)ₐ does not include the sequence Gly-Pro.

2. A DNA construct according to Claim 1 wherein (Z)ₐ is a chain of two or three amino acids, the DNA sequence for which defines a restriction endonuclease cleavage site.

3. A DNA construct according to Claim 1 wherein a is zero.

4. A DNA construct according to any one of the preceding claims in which (Z)ₐ is free from glycine and/or proline.

5. A DNA construct according to any one of the preceding claims wherein the group (Z)ₐ is a chain of amino acids substantially devoid of biological activity.

6. A DNA construct according to any one of the preceding Claims wherein the heterologous protein Het is an antigenic sequence derived from a virus, bacterium, fungus, yeast or parasite.

7. A DNA construct according to Claim 6 wherein the heterologous protein Het is the Schistosoma mansoni P28 glutathione *S*-transferase antigen.

8. A replicable expression vector containing a DNA construct as defined in any one of the preceding claims.

9. A replicable expression vector according to claim 8 suitable for use in bacteria.

10. A NON-HUMAN host having integrated into the chromosomal DNA thereof a DNA construct as defined in any one of Claims 1 to 7.

11. A host according to claim 10 which is a bacterium.

12. A fusion protein encoded by a DNA construct as defined in any one of Claims 1 to 7.

13. A process for the preparation of a bacterium which process comprises transforming a bacterium with a DNA construct as defined in any one of Claims 1 to 7.

14. A process according to claim 13 wherein the bacterium is an attenuated bacterium.

15. A vaccine composition comprising a fusion protein as defined in claim 12 and a pharmaceutically acceptable carrier.

16. A vaccine composition comprising an attenuated bacterium expressing a fusion protein encoded by a DNA construct as defined in any one of claims 1 to 7; and a pharmaceutically acceptable carrier.

## Patentansprüche

1. DNA-Konstrukt, umfassend eine DNA-Sequenz, die für ein Fusionprotein der Formel TetC-(Z)ₐ-Het kodiert, worin TetC das C-Fragment des Tetanus-Toxins ist, Het ein heterologes Protein ist, Z eine Aminosäure ist und a kleiner 4 ist, vorausgesetzt, dass (Z)ₐ nicht die Sequenz Gly-Pro enthält.

2. DNA-Konstrukt nach Anspruch 1, worin (Z)ₐ eine Kette von zwei oder drei Aminosäuren ist, wobei die DNA-Sequenz dafür eine Restriktionsendonuklease-Spaltstelle definiert.

3. DNA-Konstrukt nach Anspruch 1, worin a Null ist.

4. DNA-Konstrukt nach einem der vorangehenden Ansprüche, worin (Z)ₐ frei von Glycin und/oder Prolin ist.

5. DNA-Konstrukt nach einem der vorangehenden Ansprüche, worin die Gruppe (Z)ₐ eine Kette von Aminosäuren ist, die im wesentlichen frei von biologischer Aktivität ist.

6. DNA-Konstrukt nach einem der vorangehenden Ansprüche, worin das heterologe Protein Het eine antigene Sequenz ist, die von einem Virus, Bakterium, Fungus, Hefe oder Parasiten stammt.

7. DNA-Konstrukt nach Anspruch 6, worin das heterologe Protein Het das Schistosoma mansoni-P28-Glutathion-S-transferase-Antigen ist.

8. Replizierbarer Expressionsvektor, enthaltend ein DNA-Konstrukt, wie in einem der vorangehenden Ansprüche definiert.

9. Replizierbarer Expressionsvektor nach Anspruch 8, der sich zur Anwendung in Bakterien eignet.

10. Nicht-humaner Wirt, in dessen chromosomale DNA ein DNA-Konstrukt integriert ist, wie in einem der Ansprüche 1 bis 7 definiert.

11. Wirt nach Anspruch 10, der ein Bakterium ist.

12. Fusionsprotein, das durch ein DNA-Konstrukt kodiert ist, wie in einem der Ansprüche 1 bis 7 definiert.

13. Verfahren zur Präparierung eines Bakteriums, welches Verfahren das Transformieren eines Bakteriums mit einem DNA-Konstrukt umfasst, wie in einem der Ansprüche 1 bis 7 definiert.

14. Verfahren nach Anspruch 13, wobei das Bakterium ein attenuiertes Bakterium ist.

15. Vakzine-Zusammensetzung, umfassend ein Fusionsprotein, wie in Anspruch 12 definiert, und einen pharmazeutisch geeigneten Träger.

16. Vakzine-Zusammensetzung, umfassend ein attenuiertes Bakterium, das ein Fusionsprotein exprimiert, welches durch ein DNA-Konstrukt kodiert ist, wie in einem der Ansprüche 1 bis 7 definiert; und einen pharmazeutisch geeigneten Träger.

## Revendications

1. Construction d'ADN comprenant une séquence d'ADN codant une protéine de fusion de formule TetC-(Z)ₐ-Het, dans laquelle TetC est le fragment C de la toxine tétanique, Het est une protéine hétérologue, Z est un acide aminé, et a est inférieur à 4, à condition que (Z)ₐ n'inclut pas la séquence Gly-Pro.

2. Construction d'ADN selon la revendication 1, dans laquelle (Z)ₐ est une chaîne de deux ou trois acides aminés, dont la séquence d'ADN définit un site de clivage par endonucléase de restriction.

3. Construction d'ADN selon la revendication 1, dans laquelle a vaut zéro.

4. Construction d'ADN selon l'une quelconque des revendications précédentes, dans laquelle (Z)ₐ est dépourvu de résidu de glycine et/ou de proline.

5. Construction d'ADN selon l'une quelconque des revendications précédentes, dans laquelle le groupe (Z)ₐ est une chaîne d'acides aminés sensiblement dépourvue d'activité biologique.

6. Construction d'ADN selon l'une quelconque des revendications précédentes, dans laquelle la protéine hétérologue Het est une séquence antigénique dérivée d'un virus, d'une bactérie, d'un champignon, d'une levure ou d'un parasite.

7. Construction d'ADN selon la revendication 6, dans laquelle la protéine hétérologue Het est l'antigène glutathion *S*-transférase P28 de Schistosoma mansoni.

8. Vecteur d'expression réplicable contenant une construction d'ADN telle que définie dans l'une quelconque des revendications précédentes.

9. Vecteur d'expression réplicable selon la revendication 8, convenant à une utilisation dans des bactéries.

10. Hôte non humain ayant, intégré dans son ADN chromosomique, une construction d'ADN telle que définie dans l'une quelconque des revendications 1 à 7.

11. Hôte selon la revendication 10, qui est une bactérie.

12. Protéine de fusion codée par une construction d'ADN telle que définie dans l'une quelconque des revendications 1 à 7.

13. Procédé pour la préparation d'une bactérie, ce procédé comprenant le fait de transformer une bactérie avec une construction d'ADN telle que définie dans l'une quelconque des revendications 1 à 7.

14. Procédé selon la revendication 13, dans lequel la bactérie est une bactérie atténuée.

15. Composition de vaccin comprenant une protéine de fusion telle que définie dans la revendication 12 et un véhicule pharmaceutiquement acceptable.

16. Composition de vaccin comprenant une bactérie atténuée exprimant une protéine de fusion codée par une construction d'ADN telle que définie dans l'une quelconque des revendications 1 à 7 ; et un véhicule pharmaceutiquement acceptable.
